(19) **Europäisches Patentamt**

**European Patent Office**

**Office européen des brevets**

(11) **EP 0 584 408 B1**

(12) **EUROPEAN PATENT SPECIFICATION**

(45) Date of publication and mention
of the grant of the patent:
**07.01.1999 Bulletin 1999/01**

(51) Int. Cl.$^6$: **C07D 315/00**

(21) Application number: **92202909.5**

(22) Date of filing: **22.09.1992**

(54) **Process for the preparation of gamma-butyrolactone**

Verfahren zur Herstellung von Gamma-Butyrolacton

Procédé pour la préparation de gamma-butyrolactone

(84) Designated Contracting States:
**AT DE ES FR GB IT SE**

(30) Priority: **25.08.1992 JP 248560/92**

(43) Date of publication of application:
**02.03.1994 Bulletin 1994/09**

(73) Proprietor:
**Tonen Chemical Corporation**
**Chuo-Ku Tokyo (JP)**

(72) Inventors:
• **Ichiki, Tatsumi**
**Iruma-gun, Saitama-ken (JP)**
• **Kobayashi, Kenji**
**Iruma-gun, Saitama-ken (JP)**
• **Suzuki, Sadakatsu**
**Iruma-gun, Saitama-ken (JP)**
• **Ueno, Hiroshi**
**Hiki-gun, Saitama-ken (JP)**

(74) Representative:
**Smulders, Theodorus A.H.J., Ir. et al**
**Vereenigde Octrooibureaux**
**Nieuwe Parklaan 97**
**2587 BN 's-Gravenhage (NL)**

(56) References cited:
**EP-A- 0 523 774**          **WO-A-92/00973**
**US-A- 2 420 250**

• **CHEMICAL ABSTRACTS, vol. 106, no. 17, 27**
**April 1987, Columbus, Ohio, US; abstract no.**
**138244w, page 672 ;column L ; & JP-A-61 246**
**173 (IDEMITSU PETROCHEMICAL CO)**
• **CHEMICAL ABSTRACTS, vol. 116, no. 9, 2 March**
**1992, Columbus, Ohio, US; abstract no. 83526p,**
**page 804 ;column R ; & JP-A-03 232 874 (TONEN**
**CORP)**
• **CHEMICAL ABSTRACTS, vol. 116, no. 17, 27**
**April 1992, Columbus, Ohio, US; abstract no.**
**173996, page 847 ;column L ; & JP-A-03 232 875**
**(TONEN CORP)**

Printed by Xerox (UK) Business Services
2.16.7/3.6

**Description**

FIELD OF THE INVENTION

The present invention relates to a process for the preparation of $\gamma$-butyrolactone by catalytically dehydrogenating 1,4-butanediol in a gaseous phase in the presence of a catalyst, which process allows recycling of unreacted 1,4-butanediol.

PRIOR ART

Gamma-butyrolactone is a useful compound as a solvent or an intermediate product for the preparation of pyrrolidones such as N-methyl pyrrolidone. Therefore, there is an eager need for a less costly and efficient process for the preparation of $\gamma$-butyrolactone.

Known processes for the preparation of $\gamma$-butyrolactone include a method where 1,4-butanediol is dehydrogenated in the presence of copper-chromium catalysts. The present inventors found that when manganese and/or barium, and sodium and/or potassium are added to a copper-chromium catalyst, activity and selectivity of the catalyst are enhanced and the life of the catalyst is prolonged and that when this catalyst is reduced in specific conditions, the activity of the catalyst is further increased (copending U.S. Patent Application Serial No. 07/915,076, European Patent Application No. 92201986.4).

In commercial production of $\gamma$-butyrolactone, recycling of unreacted 1,4-butanediol remaining in a reaction mixture is advantageous for reduction of production costs. In the aforesaid method, dehydrated dimmers such as 2-(4-hydroxybutoxy)-tetrahydrofuran (hereinafter referred to as HBTHF) and 2-(4-oxobutoxy)-tetrahydrofuran (hereinafter, OBTHF) are formed as by-products.

It is known that in a process for separation of butanediols a mixture of 2-methyl-1,3-propanediol and 1,4-butanediol accompanied with HBTHF as an impurity is heated in the presence of a ruthenium catalyst, water and hydrogen to convert HBTHF into 1,4-butanediol, where other noble metals than ruthenium, nickel and cobalt are said to be ineffective (Japanese Patent Application Laid-Open No. Sho-58-167532/83). It is also known that even if crude 1,4-butanediol containing HBTHF and OBTHF is distilled in a process for the preparation of 1,4-butanediol, such by-products form an azeotrope with 1,4-butanediol to make it difficult to purify 1,4-butanediol and, therefore, a purification method is proposed where the crude 1,4-butanediol is hydrogenated in the presence of a hydrogenation catalyst; and only platinum group elements such as metallic palladium and platinum are described as the catalyst (Japanese Patent Application Laid-Open No. Sho-61-197534/86).

If the aforesaid methods for the purification of 1,4-butanediol butanediol are used in a process for the preparation of $\gamma$-butyrolactone to purify the unreacted reactant, the process will be too much complicated and suffer from increased costs.

The international patent application WO-A-92/00973 is concerned with a process for dehydrogenating diols. In the examples, a process for preparing $\gamma$-butyrolactone from 1,4-butanediol in the liquid phase using a copper chromite catalyst in the absence of added hydrogen is described.

In the US patent 2,420,250, a process for preparing $\gamma$-valerolactone is disclosed, wherein 1,4-pentanediol is heated in the presence of a copper chromite catalyst until evolution of hydrogen gas has ceased. The European patent application 0 523 774 describes a process for catalytically dehydrogenating 1,4-butanediol in a gaseous phase, using a catalyst comprising, apart from copper, chromium, and manganese and/or barium, sodium and/or potassium.

SUMMARY OF THE INVENTION

A purpose of the invention is to provide a process for the preparation of $\gamma$-butyrolactone which process is less costly and efficient and allows recycling of the unreacted.

The present inventors have now found that even if a bottom in distillation of $\gamma$-butyrolactone from a reaction mixture, which bottom contains unreacted 1,4-butanediol as well as impurities, is recycled as such in a feed in a process for the preparation of $\gamma$-butyrolactone by catalytically dehydrogenating 1,4-butanediol in a gaseous phase in the presence of hydrogen using a copper-chromium catalyst, the activity, selectivity or life of the catalyst is not adversely affected at all and, in addition, that HBTHF and OBTHF may be decomposed even with a copper-chromium catalyst and may serve as a reactant for the product.

Thus, the present invention is a process for the preparation of $\gamma$-butyrolactone by catalytically dehydrogenating 1,4-butanediol in a gaseous phase in the presence of a catalyst, characterized in that reaction is carried out in the presence of a catalyst containing copper and chromium and in the presence of hydrogen, and $\gamma$-butyrolactone formed is separated from a reaction mixture providing a residual part comprising 1,4-butanediol and 2-(4-hydroxybutoxy)-tetrahydrofuran and 2-(4-oxobutoxy)-tetrahydrofuran, which is recycled in a feed.

BRIEF DESCRIPTION OF THE DRAWINGS

Figures 1 and 2 are flow charts to demonstrate embodiments of the invention.
Figure 3 is a graph to show how HBTHF affects catalyst life.

DESCRIPTION OF THE PREFERRED EMBODIMENT

The catalyst used in the process of the invention may be any known copper-chromium catalysts. However, the catalyst preferably contains manganese and/or barium, and sodium and/or potassium in addition to copper and chromium as described in copending U.S. Patent Application Serial No. 07/915,076, or European Patent Application No. 92201986.4. The other alkali metals (e.g., lithium, rubidium and cesium) than sodium and potassium are not suitable for use. An atomic ratio of copper to chromium is preferably 0.4 to 1.8, more preferably 0.8 to 1.4. Manganese is contained preferably in an amount of 1 to 10 parts by weight, more preferably 2 to 7 parts by weight, per 100 parts by weight of the total copper and chromium. Barium is contained preferably in an amount of 2 to 20 parts by weight, more preferably 2 to 10 parts by weight, per 100 parts by weight of the total of copper and chromium. When both manganese and barium are contained, their total is preferably 3 to 30 parts by weight per 100 parts by weight of the total of copper and chromium. Sodium and potassium is contained preferably in an amount of 0.1 to 10 parts by weight, more preferably 0.5 to 7 parts by weight, as alkali metals, per 100 parts by weight of the total of copper and chromium. In addition, a small amount of silicon may be contained up to 10 parts by weight per 100 parts by weight of the total of copper and chromium.

The above catalyst may be prepared as follows: copper nitrate, copper sulfate, copper chloride or copper acetate may be used as a copper source; dichromate (e.g., $Na_2Cr_2O_7$), chromate or chromium nitrate as a chromium source; barium chloride, barium nitrate, manganese chloride, manganese nitrate and manganese acetate as a manganese or barium source; and carbonate, silicate (water glass) or hydroxide as a sodium or potassium source. A chromium-containing solution is made basic by ammonium and admixed with a solution containing copper, and manganese and/or barium to form precipitates.

The precipitates thus obtained are filtrated, washed with water, dried, and then pyrolyzed at 300 to 400 °C. The powder obtained is washed with an aqueous dilute acid solution, washed with water and dried. To the catalyst precursor obtained is added a compound containing sodium and/or potassium, dried and calcined at 400 to 500 °C. Timing of the addition of sodium and/or potassium is not particularly limited, but these alkali metals are highly water-soluble and, therefore, are usually added after the aforesaid precipitates formed are washed with water and dried, or after the subsequent calcination. Then, molding aids such as graphite may be added if needed, and a predetermined shape is formed using a molding machine. Each component exists in the form of oxide in the resultant catalyst.

Reduction of the catalyst may be carried out for instance as follows: nitrogen gas which generally contains about 3 % by volume of hydrogen is passed to the catalyst at a gas hourly space velocity (G.H.S.V.), reduced at normal temperature and pressure, of 4000 to 8000 hr$^{-1}$ under pressure of several kilograms per cm$^2$ in gauge, heated at 140 to 160 °C until heat generation is not observed any more in the catalyst bed; then the hydrogen concentration and the temperature are gradually raised, and hydrogen of 100 % by volume is passed at a catalyst bed temperature of 200 °C for several hours.

In a preferred embodiment, the catalyst is subjected to the following reduction treatment. That is, (1) while an inert gas which contains 0.1 to 1 % by volume of hydrogen is passed as a reducing gas, the catalyst is preheated from a temperature below 40 °C to a temperature of from 100 to 140 °C (pre-reduction temperature), and then (2) the temperature and the hydrogen concentration are gradually raised. It is known from JP Application Laid-Open No. Hei-1-127042/89 that such reduction treatment enhances activity of copper-chromium catalyst in the preparation of 1,4-butanediol by hydrogenation of maleic diester. This treatment also has an effect of increasing activity of the Cu-Cr-Mn and/or Ba-Na and/or K catalyst in the preparation of γ-butyrolactone by dehydrogenation of 1,4-butanediol.

A gas mixture comprising 1,4-butanediol and hydrogen may be contacted with the catalyst in any manner which may be properly selected among conventional ones, for instance, in a fixed bed, in a moving bed or in a fluidized bed. Alternatively, the gas mixture may be contacted with the catalyst batchwise.

The dehydrogenation is preferably conducted at a reaction temperature of 150 to 300 °C, a reaction pressure of 0 to 8 kg/cm$^2$ in gauge pressure, a mole ratio of hydrogen to 1,4-butanediol of 0.5 to 10 and a weight hourly space velocity of 1,4-butanediol of 0.2 to 16 hour$^{-1}$.

In the present invention, γ-butyrolactone formed is recovered from a reaction mixture, and then the remaining part which contains unreacted 1,4-butanediol is recycled in a feed. This is most conveniently done by distillating a reaction mixture to distil low-boiling γ-butyrolactone away and recycling a residual bottom as such. According to the invention, a fraction containing 1,4-butanediol as well as HBTHF and OBTHF with no higher molecular weight by-products or heavier products is recycled. This may be done by distillating the distillation bottom after recover of γ-butyrolactone and recycling a low-boiling fraction containing 1,4-butanediol and azeotropic HBTHF and OBTHF. Alternatively, this is more conveniently done by drawing a middle fraction containing 1,4-butanediol from a middle part of a γ-butyrolactone distillation column, and recycling it. In another embodiment, a distillation bottom is recycled in an evaporator for a feed 1,4-butanediol and then HBTHF and OBTHF evaporate as an azeotrope with 1,4-butanediol, while heavier materials accumulate in the evaporator, which may be drawn off properly.

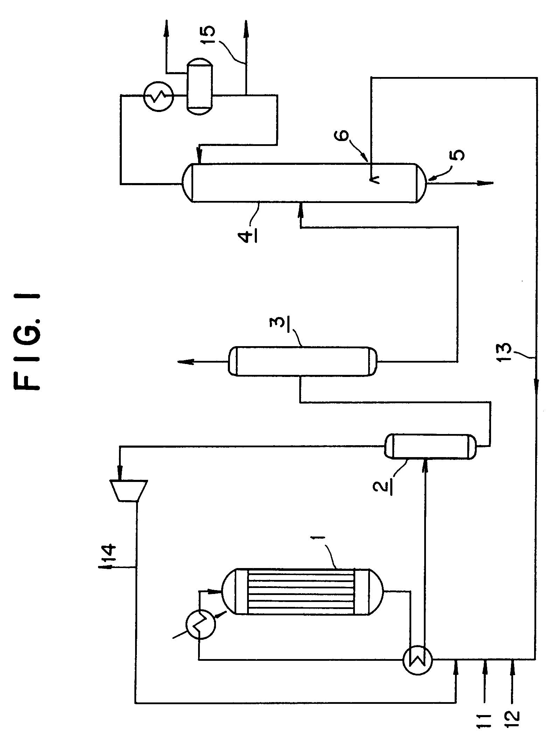

One embodiment is demonstrated in Figure 1. Catalyst is placed in reactor 1. 1,4-Butanediol is fed via conduit 11, evaporated and introduced into reactor 1. At start of operations, hydrogen gas is introduced via 12. In stationary operations, hydrogen generates and, therefore, excess hydrogen is removed from the system. A reaction mixture is transferred to gas-liquid separator 2 where hydrogen gas is separated, a part of which is recycled and excess hydrogen gas is removed via conduit 14 from the system. Meanwhile, a liquid part is sent to crude tower 3 where low-boiling materials are removed, and then to product tower 4. In the product tower, an object product γ-butyrolactone is drawn from the top and condensed, a part of which is refluxed and the remaining part is taken out via conduit 15. A side stream containing 1,4-butanediol and azeotropic impurities is drawn away from a middle part 6 of the tower, and recycled via conduit 13. Heavier products are taken out from bottom 5 and separately disposed of.

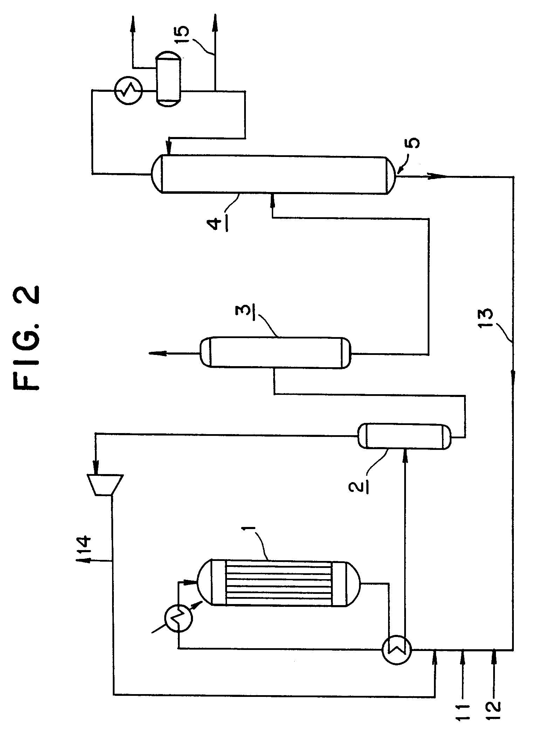

Alternatively, as shown in Figure 2, a bottom containing 1,4-butanediol is drawn from bottom 5 and recycled as such.

As decsribed above. unreacted 1.4-butanediol which contains impurities may be recycled as such without purification according to the invention. This is possible because the impurities do not adversely affect the activity, selectivity or life of the copper-chromium catalyst and because HBTHF and OBTHF are decomposed by the copper-chromium catalyst in the reaction system and, therefore, do not accumulate in the system when recycled.

The present invention will be further explained with reference to the following unlimitative examples.

Example 1

This example is to demonstrate how impurities affect activity and selectivity of a catalyst when unreacted 1,4-butanediol containing impurities is recycled. A model feed 1,4-butanediol used contained HBTHF, i.e., 2-(4-hydroxybutoxy)-tetrahydrofuran, which was contacted with a Cu-Cr-Mn-Ba-Na catalyst to produce γ-butyrolactone.

(1) Synthesis of HBTHF

Dihydrofuran (DHF) was reacted with 1,4-butanediol at a mole ratio of 1/10 using a catalyst Amberlyst 16 (Rohm and Haas Japan Company). The product was extracted with benzene, and the benzene was then flash distilled. The residue was subjected to distillation to obtain a distillate containing highly concentrated HBTHF. The composition of the distillate was as follows: 88% by weight of HBTHF, 7.5 % by weight of 1,4-butanediol and 3.5 % by weight of 1,4-di-(2'-tetrahydrofuroxy)butane.

(2) Preparation of a catalyst

A catalyst used was prepared and reduced as follows:

(a) Preparation of a catalyst

To a solution of 150 g of $Na_2Cr_2O_7 \cdot 2H_2O$ in 900 ml of distilled water were added 225 ml of an aqueous 28 % ammonia, which is hereinafter referred to as Solution A.

In 900 ml of distilled water were dissolved 280 g of $Cu(NO_3)_2 \cdot 3H_2O$, 26 g of $Mn(NO_3)_2 \cdot 6H_2O$ and 8 g of $Ba(NO_3)_2$, and heated to 80 °C. This solution is hereinafter referred to as Solution B.

Solution B was added to Solution A under stirring. The resultant precipitates were filtrated, washed with water, dried and pulverized, which was then pyrolyzed at 350 °C. The powder obtained was washed with an aqueous 10 % solution of acetic acid, washed with water and dried to obtain a catalyst precursor. To 100 g of the catalyst precursor were added 15 g of sodium silicate (water glass No. 1), dried and then calcined at 450 °C for 3 hours, to which graphite was added in an amount of 0.5 % by weight. This was molded into pellets. The composition of the catalyst obtained in an oxide form which was determined in fluorescence X-ray analysis was as follows: 28.4 % by weight of Cu, 25.0 % by weight of Cr, 2.3 % by weight of Mn, 1.6 % by weight of Ba and 1.7 % by weight of Na.

(b) Reduction of the catalyst

Ten ml of the catalyst in an oxide form prepared above were packed in a stainless steel (SUS 316) fixed bed reactor with an inner diameter of 15 mm and a length of 600 mm. The atmosphere in the system was sufficiently purged with a flow of nitrogen, which was then pressurized to 3 kg/cm²G. A flow rate of nitrogen was 15 liters/hr (gas hourly space velocity of 1500 hr$^{-1}$).

Then, while maintaining the pressure and the gas flow rate, the nitrogen gas was replaced with nitrogen containing 0.1 % by volume of hydrogen at room temperature and, subsequently, heating was started. The temperature was elevated at a rate of 30 °C/hr up to 120 °C.

After confirmed at 120 °C that the concentration of hydrogen at the gas outlet was identical with that at the gas inlet, the temperature was elevated to 130 °C over one hour. The temperature was then elevated from 130 °C to 140 °C over further one hour, from 140 °C to 150 °C over further one hour, and from 150 °C to 160 °C over further one hour, provided that the temperature was elevated after holding the predetermined temperature in each step until the concentration of hydrogen at the gas outlet became identical with that at the gas inlet.

Then, the concentration of hydrogen was increased gradually from 0.1 % by volume to 0.3 % by volume, and maintained for 2 hours.

The concentration of hydrogen was further increased gradually from 0.3 % by volume to 0.5 % by volume, and then the reduction temperature was elevated from 160 °C to 170 °C over one hour. When the temperature reached 170 °C, the concentration of hydrogen was increased to 2.0 % by volume and then held for one hour.

Subsequently, the concentration of hydrogen was increased stepwise from 2.0 % by volume to 5.0 % by volume, 10.0 % by volume and 100 % by volume. In each step, the hydrogen concentration was held constant for 1 or 2 hours. When the concentration of hydrogen reached 100 % by volume, the reduction temperature was elevated to 200 °C to end the reduction treatment. In each step, a next step was started after confirmed that the hydrogen concentration at the gas outlet was identical with that at the gas outlet.

Through the whole procedure of the reduction treatment in the above, temperature difference between the catalyst bed and the heating apparatus, $\Delta T$, due to heat generation of the catalyst bed did not exceed 5 °C.

(3) Preparation of $\gamma$-butyrolactone with a feed 1,4-butanediol containing HBTHF

1,4-Butanediol containing zero mole %, 0.26 mole %, 0.40 mole % or 0.81 mole % of HBTHF prepared in the above (1) was used as a feed. Through the catalyst reduced in the above (b), hydrogen gas and the feed were passed in reaction conditions of 230 °C, atmospheric pressure, a weight hourly space velocity (W.H.S.V.) of 1,4-butanediol of 2.0 or 5.0 $_{hour}$-1, and a molar ratio of $H_2$ to the feed of 6.0 to conduct dehydrogenation reaction. The reaction mixture were analyzed in gas chromatography to obtain the following results in mole percent. In Table 1, the conversion of BDO and the yield of GBL are defined as follows:

Conversion of BDO = 100 - (moles of BDO contained in a reaction mixture/total moles of compounds contained in a reaction mixture ) x 100

Yield of GBL = (moles of GBL contained in a reaction mixture/total moles of compounds contained in a reaction mixture) x 100

Table 1

| HBTHF content in the feed, mole % | W.H.S.V. hr⁻¹ | Conversion of BDO, % | Yield of GBL, % | Selectivity, % | | | | | | | | HBTHF content in the reaction mixture, mole% |
|---|---|---|---|---|---|---|---|---|---|---|---|---|
| | | | | Lights | THF | Butanol | BAC | HBA | GBL | HBTHF | Heavies | |
| 0 | 2 | 99.98 | 99.10 | 0.08 | 0.16 | 0.34 | 0.17 | – | 99.13 | 0.11 | 0.02 | 0.11 |
| 0.26 | 2 | 99.97 | 98.97 | 0.11 | 0.16 | 0.35 | 0.16 | – | 99.00 | 0.17 | 0.06 | 0.17 |
| 0.40 | 2 | 99.98 | 98.99 | 0.10 | 0.16 | 0.33 | 0.16 | – | 99.01 | 0.20 | 0.04 | 0.20 |
| 0.81 | 2 | 99.97 | 98.63 | 0.15 | 0.19 | 0.33 | 0.14 | – | 98.66 | 0.44 | 0.09 | 0.44 |
| 0 | 5 | 97.94 | 97.15 | 0.05 | 0.09 | 0.14 | 0.10 | 0.13 | 99.19 | 0.19 | 0.11 | 0.19 |
| 0.26 | 5 | 98.72 | 97.78 | 0.07 | 0.14 | 0.21 | 0.11 | 0.07 | 99.06 | 0.24 | 0.10 | 0.24 |
| 0.40 | 5 | 98.68 | 97.69 | 0.09 | 0.14 | 0.20 | 0.11 | 0.07 | 99.00 | 0.30 | 0.10 | 0.29 |
| 0.81 | 5 | 98.92 | 97.54 | 0.11 | 0.15 | 0.21 | 0.12 | 0.06 | 98.61 | 0.61 | 0.14 | 0.60 |

Note: THF=Tetrahydrofuran
BAC=Butyric acid
HBA=Hydroxybutyl aldehyde
GBL=γ-Butyrolactone
HBTHF=2-(4-Hydroxybutoxy)-tetrahydrofuran

It is understood from the above Table that a conversion of 1,4-butanediol and a selectivity for γ-butyrolactone were not affected by HBTHF contained in the feed γ-butyrolactone and, thus, HBTHF had no effect on activity of the catalyst. Further, it is seen that a concentration of HBTHF in the reaction mixture was less than that in the feed γ-butyrolac-

6

tone in the all cases except the case where HBTHF content in the feed was 0 %, which indicates that HBTHF was partially decomposed into 1,4-butanediol, etc. in dehydrogenation conditions of 1,4-butanediol. This means that HBTHF would not accumulate in a system even when unreacted 1,4-butanediol containing HBTHF is recycled in a feed.

Example 2

This example is to demonstrate any effect on the life of catalyst with impurities contained in a recycled feed. The model feed was 1,4-butanediol containing HBTHF. Dehydrogenation of 1,4-butanediol was conducted using the same catalyst as that prepared and reduced in (2) of Example 1, and a feed 1,4-butanediol containing HBTHF which was prepared in (1) of Example 1. The reaction conditions were as follows: reaction temperature of 232 °C, pressure of 2 $kg/cm^2G$, a W.H.S.V. of 1,4-butanediol of 5.0 $hour^{-1}$, and a molar ratio of $H_2$ to the feed of 6.0.

The concentration of HBTHF in the feed was zero mole % in a period of from the start of the reaction to 100 hours; 0.62 mole % from 100 hours to 600 hours; and 1.02 mole % from 600 hours to 1000 hours. After 1000 hours, it was again zero mole %.

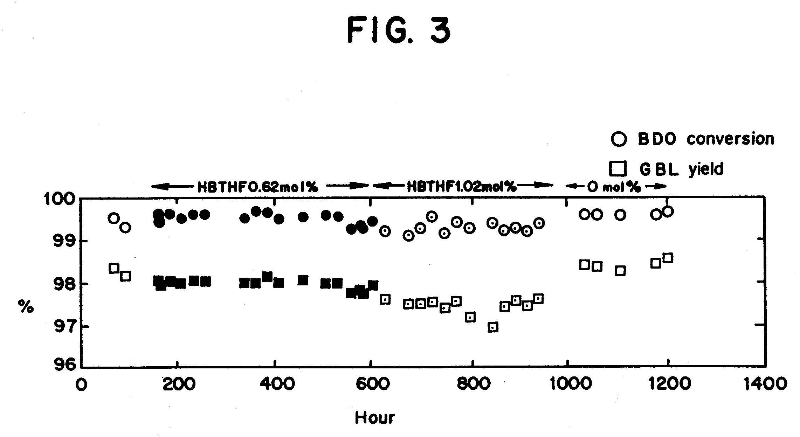

The results are as shown in Figure 3, where BDO is 1,4-butanediol and GBL is $\gamma$-butyrolactone. The conversion of 1,4-butanediol did not change with the increasing concentration of HBTHF or with the lapse of time. Although the yield of $\gamma$-butyrolactone decreased with the increasing concentration of HBTHF, the degree of the decrease in the yield corresponds to the increasing content of HBTHF in the feed. Thus, no decrease occurred in terms of a yield based on 1,4-butanediol in the feed.

When the concentration of HBTHF was again returned to zero mole % after 1000 hours, both the conversion of 1,4-butanediol and the yield for $\gamma$-butyrolactone recovered to the level seen immediately after the start of the reaction. Thus, it can be concluded that the presence of HBTHF in a feed has no effect on the life of the catalyst.

**Claims**

1. A process for the preparation of $\gamma$-butyrolactone by catalytically dehydrogenating 1,4-butanediol in a gaseous phase in the presence of a catalyst, characterized in that the reaction is carried out in the presence of a catalyst containing copper and chromium and in the presence of hydrogen, and $\gamma$-butyrolactone formed is separated from a reaction mixture providing a residual part comprising 1,4-butanediol and 2-(4-hydroxybutoxy)-tetrahydrofuran and 2-(4-oxobutoxy)-tetrahydrofuran, which is recycled in a feed.

2. The process as claimed in claim 1, wherein the formed $\gamma$-butyrolactone is distilled away from a top of a distillation tower, and a side stream containing unreacted 1,4-butanediol and azeotropic impurities is drawn from the tower and recycled in a feed.

3. The process as claimed in claim 1, wherein the formed $\gamma$-butyrolactone is distilled away from a top of a distillation tower, and a bottom containing unreacted 1,4-butanediol and impurities is recycled in a feed.

4. The process as claimed in claim 1, wherein the catalyst contains copper, chromium, and at least one member selected from the group consisting of manganese and barium, and further at least one member selected from the group consisting of sodium and potassium.

5. The process as claimed in claim 4, wherein an atomic ratio of copper to chromium is 0.4 to 1.8.

6. The process as claimed in claim 4, wherein the catalyst contains manganese in an amount of 1 to 10 parts by weight per 100 parts by weight of the total of copper and chromium.

7. The process as claimed in claim 4, wherein the catalyst contains barium in an amount of 2 to 20 parts by weight per 100 parts by weight of the total of copper and chromium.

8. The process as claimed in claim 4, wherein the catalyst contains both manganese and barium in a total amount of 3 to 30 parts by weight per 100 parts by weight of the total of copper and chromium.

9. The process as claimed in claim 1 or 4, wherein the dehydrogenation is conducted at a reaction temperature of 150 to 300°C, a reaction pressure of 0 to 8 $kg/cm^2$ in gauge pressure, a mole ratio of hydrogen to 1,4-butanediol of 0.5 to 10, and a weight hourly space velocity of 1,4-butanediol of 0.2 of 16 $hour^{-1}$.

**Patentansprüche**

1.  Verfahren zur Herstellung von γ-Butyrolacton durch katalytische Dehydrierung von 1,4-Butandiol in Gasphase und Gegenwart eines Katalysators, dadurch gekennzeichnet, daß die Reaktion in Gegenwart eines Kupfer und Chrom enthaltenden Katalysators und in Gegenwart von Wasserstoff durchgeführt wird und daß das gebildete γ-Butyrolacton von einem Reaktionsgemisch abgetrennt wird, wobei ein 1,4-Butandiol und 2-(4-Hydroxybutoxy)tetrahydrofuran sowie 2-(4-Oxobutoxy)tetrahydrofuran umfassender Teil zurückbleibt, der wieder zugeführt wird.

2.  Verfahren nach Anspruch 1, bei dem das gebildete γ-Butyrolacton vom oberen Ende eines Destillationsturms abdestilliert wird und ein Seitenstrom, der nicht umgesetztes 1,4-Butandiol und azeotrope Verunreinigungen enthält, vom Turm abgezogen und wieder zugeführt wird.

3.  Verfahren nach Anspruch 1, bei dem das gebildete γ-Butyrolacton vom oberen Ende eines Destillationsturms abdestilliert wird und ein Bodenstrom, der nicht umgesetztes 1,4-Butandiol und Verunreinigungen enthält, wieder zugeführt wird.

4.  Verfahren nach Anspruch 1, bei dem der Katalysator Kupfer, Chrom und mindestens eine Komponente aus der Gruppe Mangan und Barium sowie außerdem mindestens eine Komponente aus der Gruppe Natrium und Kalium enthält.

5.  Verfahren nach Anspruch 4, bei dem ein Atomverhältnis von Kupfer zu Chrom 0,4 zu 1,8 beträgt.

6.  Verfahren nach Anspruch 4, bei dem der Katalysator Mangan in einer Menge von 1 bis 10 Gewichtsteilen auf 100 Gewichtsteile der Gesamtmenge von Kupfer und Chrom enthält.

7.  Verfahren nach Anspruch 4, bei dem der Katalysator Barium in einer Menge von 2 bis 20 Gewichtsteilen auf 100 Gewichtsteile der Gesamtmenge von Kupfer und Chrom enthält.

8.  Verfahren nach Anspruch 4, bei dem der Katalysator sowohl Mangan als auch Barium in einer Gesamtmenge von 3 bis 30 Gewichtsteilen auf 100 Gewichtsteile der Gesamtmenge von Kupfer und Chrom enthält.

9.  Verfahren nach Anspruch 1 oder 4, bei dem die Dehydrierung bei einer Reaktionstemperatur von 150 bis 300°C, einem Reaktionsdruck von 0 bis 8 kg/cm$^2$G, einem Molverhältnis von Wasserstoff zu 1,4-Butandiol von 0,5 bis 10 und einem gewichtsbezogenen Durchsatz von 1,4-Butandiol von 0,2 von 16 Stunden$^{-1}$ erfolgt.

**Revendications**

1.  Procédé de préparation de γ-butyrolactone par déshydrogénation catalytique du 1,4-butanediol en phase gazeuse en présence d'un catalyseur, caractérisé en ce que l'on effectue la réaction en présence d'un catalyseur contenant du cuivre et du chrome et en présence d'hydrogène, et en ce qu'on sépare la γ-butyrolactone formée du mélange de réaction pour obtenir une partie résiduelle comprenant du 1,4-butanediol, du 2-(4-hydroxybutoxy)tétrahydrofurane et du 2-(4-oxobutoxy)tétrahydrofurane, que l'on recycle

2.  Procédé selon la revendication 1, dans lequel on sépare par distillation la γ-butyrolactone formée en haut d'une colonne de distillation et on soutire de la colonne une fraction latérale contenant du 1,4-butanediol n'ayant pas réagi et des impuretés azéotropiques que l'on recycle.

3.  Procédé selon la revendication 1, dans lequel on sépare par distillation la γ-butyrolactone formée en haut d'une colonne de distillation et on recycle le résidu de distillation contenant du 1,4-butanediol n'ayant pas réagi et les impuretés

4.  Procédé selon la revendication 1, dans lequel le catalyseur contient du cuivre, du chrome et au moins un élément choisi dans le groupe constitué par le manganèse et le baryum, et en outre au moins un autre élément choisi dans le groupe constitué par le sodium et le potassium.

5.  Procédé selon la revendication 4, dans lequel le rapport atomique du cuivre au chrome est de 0,4 à 1,8.

6.  Procédé selon la revendication 4, dans lequel le catalyseur contient du manganèse en une quantité de 1 à 10 parties en poids pour 100 parties en poids de la quantité totale de cuivre et de chrome.

7. Procédé selon la revendication 4, dans lequel le catalyseur contient du baryum en une quantité de 2 à 20 parties en poids pour 100 parties en poids de la quantité totale de cuivre et de chrome.

8. Procédé selon la revendication 4, dans lequel le catalyseur contient à la fois du manganèse et du baryum en une quantité totale de 3 à 30 parties en poids pour 100 parties en poids de la quantité totale de cuivre et de chrome.

9. Procédé selon la revendication 1 ou 4, dans lequel on effectue la déshydrogénation à une température de réaction de 150 à 300°C, à une pression de réaction de 0 à 8 kg/cm$^2$ au manomètre, à un rapport molaire de l'hydrogène au 1,4-butanediol de 0,5 à 10, et à une vitesse spatiale horaire pondérale du 1,4-butanediol de 0,2 à 16 heures$^{-1}$.

FIG. I

FIG. 2

# FIG. 3

EP 0 584 408 B1